# EUROPÄISCHE PATENTSCHRIFT

(11) **EP 3 150 120 B1**
(45) Veröffentlichungstag und Bekanntmachung des Hinweises auf die Patenterteilung: **03.10.2018**
(21) Anmeldenummer: 16195115.7
(22) Anmeldetag: 10.07.2008
(51) Int. Cl.: A61B 5/0408, A61F 2/78, A61N 1/04, A61F 2/00, A61F 2/70, A61F 2/80, D04B 1/26, A61F 2/72, A61B 5/00, A61F 2/30, A61F 2/50

(54) **ORTHOPÄDISCHES INTERFACE**
ORTHOPEDIC INTERFACE
INTERFACE ORTHOPÉDIQUE

(30) Priorität: 26.07.2007 DE 102007035409
(43) Veröffentlichungstag der Anmeldung: 05.04.2017
(62) Teilanmeldung aus: 08784310.8
(73) Patentinhaber: Otto Bock HealthCare GmbH, 37115 Duderstadt (DE)
(72) Erfinder: Kettwig, Thomas, 23923 Lüdersdorf (DE); Zarling, Sven, 37115 Duderstadt (DE); Garus, Bernard, 37574 Einbeck (DE); Ruess, Felix, 2201 Gerasdorf (AT)
(74) Vertreter: Lins, Martina

(56) Entgegenhaltungen:
- WO-A-2005/052235
- WO-A2-2007/051122
- US-A- 5 413 611
- US-A- 5 443 525
- US-A1- 2006 089 725
- US-B1- 6 499 320

## Beschreibung

Die Erfindung betrifft ein orthopädisches Interface mit einem flächigen 3D-Textil mit einer Oberseite und einer Unterseite, die über Stützfäden zueinander beabstandet gehalten und miteinander verbunden sind, wobei die Unterseite des 3D-Textils zur Auflage auf der Haut eines Interfacenutzers ausgebildet ist, sowie ein System aus einem orthopädischen Interface und einer Orthesen- oder Protheseneinrichtung.
Aus der DE 102 19 814 B4 ist eine physiologische Manschette zum Anlegen an eine menschliche Extremität bekannt, die im Tragezustand gedehnt und der Körperform angepasst ist. Unter einem bestimmten Druck komprimiert die angelegte Manschette das muskuläre Gewebe, wobei vorgesehen ist, dass die Manschette aus einem Abstandsgewirk mit elastischen Fäden besteht. Durch Anlegen der Manschette wird eine erhöhte Durchblutung des muskulären Gewebes im Ruhezustand bewirkt. Die Manschette dient zur Verbesserung der Fitness des Trägers und nicht als orthopädisches Interface.
Weiterhin sind aus dem Stand der Technik so genannte Liner aus Silikon oder einem Copolymer bekannt, die über einem Amputationsstumpf getragen werden, um eine gepolsterte und luftdichte Kopplung zwischen einem Prothesenaußenschaft und einem Amputationsstumpf herzustellen. Ebenfalls sind Liner aus Polyurethan bekannt, die unmittelbar auf der Haut des Interfacenutzers getragen werden.
Die WO 1007/051122 A1 betrifft ein 3D-Textil zur Auflage auf eine Gliedmaße. An der Außenseite des 3D-Textils wird Gips oder ein Gipsverband aufgebracht.
Die US 2006/089725betrifft einen Liner aus einem elastischen, elektrisch isolierenden Material, in dem ein Hohlraum zur Aufnahme eines Stumpfes ausgebildet ist. Eine den hülsenförmigen Körper umgebende Schicht ist aus einem elektrisch leitenden Material hergestellt.

Die US 2006/089725 A1 betrifft einen Liner aus einem elastischen, elektrisch isolierenden Material, in dem ein Hohlraum zur Aufnahme eines Stumpfes ausgebildet ist. Eine den hülsenförmigen Körper umgebende Schicht ist aus einem elektrisch leitenden Material hergestellt.

Die US 6,499,320 B1 betrifft ein Gewebe mit antimikrobiellen Eigenschaften zum Einsatz unterhalb eines Prothesenschaftes. Das Gewebe weist eine Innenmaterialschicht auf, die zur Oberfläche des Stumpfes hin gerichtet ist. Das Material ist aus Fäden gewebt, von denen zumindest ein Faden ein antimikrobielles Garn ist, das mit Silber beschichtet ist.

Die US 5,413,611 A betrifft eine computergesteuerte Unterarmprothese mit einem Prothesenschaft, der aus einem leichten, stabilen Material ausgebildet ist und in Größe und Form einem natürlichen Unterarm entspricht. Auf der Innenseite des Unterarmteils ist ein Elektrodenpaar angeordnet, das ein myoelektrisches Signal zur Steuerung einer motorisch angetriebenen Prothesenhand aufnimmt und an eine Steuerungseinrichtung weiterleitet.

Die WO 2005/052235 A1 betrifft ein 3D-Abstandsgewirk mit einer ersten Außenschicht, einer zweiten Außenschicht und einer dazwischen angeordneten Zwischenschicht, wobei zumindest ein Teil der Außenschichten ein Hybridgarn aufweist, wobei das Hybridgarn elektrisch leitende Materialien wie Karbonfasern, Metalldrähte oder Spulen und dergleichen aufweist. Ein eingewebtes Muster solcher Fasern oder Drähte in das Abstandsgewirk kann eine Heizung ermöglichen. Ebenfalls kann durch Einführen von Wärmeenergie eine Versteifung in dem Material bewirkt werden, beispielsweise, um eine Form eines Schaftes zu konservieren.

Die US 5,443,525 A betrifft einen Liner, der aus einem herkömmlichen Strumpfmaterial oder aus einem Silikon hergestellt sein kann. Ein Elektrodenfeld ist an der inneren Oberfläche des Liners befestigt. Innerhalb des Liners ist ein Fenster ausgebildet, um einen Zugang zu dem Elektrodenfeld von außen zu erlangen.

Aufgabe der vorliegenden Erfindung ist es, ein orthopädisches Interface sowie ein System aus Interface und einer Orthesen- oder Protheseneinrichtung bereitzustellen, mit denen eine verbesserte Tragfähigkeit und ein höherer Tragekomfort bereitgestellt werden können.

Erfindungsgemäß wird diese Aufgabe durch ein orthopädisches Interface mit den Merkmalen des Anspruchs 1 und durch ein System mit den Merkmalen des Anspruchs 13 gelöst. Vorteilhafte Ausgestaltungen und Weiterbildungen der Erfindungen sind in den Unteransprüchen aufgeführt.

Das erfindungsgemäße orthopädische Interface mit einem flächigen 3D-Textil mit einer Oberseite und einer Unterseite, die über Stützfäden zueinander beabstandet gehalten und miteinander verbunden sind und bei dem die Unterseite des 3D-Textils zur Auflage auf der Haut eines Interfacenutzers ausgebildet ist, sieht vor, dass die der Haut zugeordnete Oberfläche oder Seite der Unterseite zumindest teilweise mit einer haftenden, also auf der Haut haftenden Beschichtung versehen ist, um die Zuordnung des orthopädischen Interfaces zu dem Körperteil, beispielsweise einem Amputationsstumpf oder einer mit einer Orthese zu unterstützenden Extremität, zu gewährleisten. Durch das 3D-Textil wird ein erhöhter Tragekomfort bereitgestellt, da punktuelle Druckkräfte verteilt werden. Weiterhin sind 3D-Textilien atmungsaktiv und lassen einen natürlichen Flüssigkeitsaustausch über die Haut zu. Ebenfalls kann die Beschichtung elektrisch leitfähig ausgebildet sein, um die Hautoberfläche zu stimulieren, Potentiale abzuleiten oder um Signale, beispielsweise an der Außenseite des 3D-Textils, weiterzuleiten. Die Signalleitung kann z.B. zu Sensoren oder Auswerteeinrichtungen führen. Eine Variante der Erfindung sieht vor, dass zusätzlich oder alternativ Elektroden auf der Innenseite des Interfaces angebracht oder in dem 3D-Textil eingearbeitet sind, um Muskel- oder Nervensignale abzuleiten oder Muskeln und Nerven zu stimulieren. Die Elektroden können auf verschiedene Arten und Weisen aufgebracht werden, z.B. aufgestickt, aufgenäht, aufgeklebt oder aufgeschweißt werden.

Ebenfalls ist es vorgesehen, dass die Außenseite des orthopädischen Interfaces, also die Oberfläche der Oberseite, zumindest teilweise mit einer Beschichtung versehen ist, die eine erhöhte Haftung an dem orthopädischen Interface begünstigt. Beispielsweise kann die Beschichtung bzw. können die Beschichtungen aus Silikon, Polyurethan oder einem Copolymer bestehen, wodurch eine verbesserte Haftung einerseits auf der Haut und andererseits an weiteren orthopädischen Komponenten wie Orthesen oder Prothesen gegeben ist.

Die Beschichtung oder die Beschichtungen können in zueinander beabstandeten Bereichen auf den jeweiligen Oberflächen der Ober- und/oder Unterseiten aufgebracht werden, beispielsweise in Streifenform, punktförmig, ringförmig oder auch in anderen flächigen Ausgestaltungen. Ebenfalls ist es möglich, dass nur in besonders beanspruchten Bereichen des orthopädischen Interfaces eine entsprechende Beschichtung auf den Oberflächen aufgebracht ist, während in weniger beanspruchten Bereichen keine Beschichtung vorhanden ist. Dies erhöht den Tragekomfort und verbessert den Wärme- und Feuchtigkeitsaustausch von der Haut durch das Interface in die Umgebung. Alternativ ist es vorgesehen, dass die Beschichtung bzw. die Beschichtungen vollflächig aufgebracht sind, um einen maximalen Halt des Interfaces zu gewährleisten. Zur Beibehaltung eines Feuchtigkeitsaustausches kann die Beschichtung feuchtigkeitsdurchlässig sein. Dazu sind die Beschichtungsmaterialien entsprechend zu wählen, beispielsweise in Gestalt von Polyurethan oder einem Copolymer. Ebenfalls können großflächige Beschichtungsbereiche mit Perforationen versehen sein, um einen Luft- und Wasserdurchtritt zu ermöglichen. Selbst bei einer vollflächigen Beschichtung ist es möglich, dass ein Gas- und Feuchtigkeitsaustausch stattfindet, da sich beispielsweise dünne Silikonbeschichtungen als atmungsaktiv herausgestellt haben. Durch die zwischen der Oberseite und der Unterseite befindliche Luftschicht in dem 3D-Textil ist eine zusätzliche Wärmeisolierung gegeben.

Die verarbeiteten Fäden des 3D-Textils können für sich ebenfalls beschichtet sein, um spezielle Anforderungen an das Textil zu erfüllen. Die Beschichtungen können eine verbesserte Hautverträglichkeit oder einen verbesserten Feuchtigkeitstransport bewirken, um so den Tragekomfort des Interfaces weiter zu erhöhen.

Zur Verbesserung der Hautverträglichkeit kann die Beschichtung der Fäden oder des Interfaces mit einem antibakteriellen Mittel getränkt oder versetzt sein, ebenfalls ist es vorgesehen, dass Silberfäden in der Unterseite oder dem 3D-Textil eingewebt oder eingesponnen sind, um die Hautverträglichkeit zu verbessern. Auch kann das 3D-Textil oder die Beschichtung oder können die Beschichtungen mit Silber bedampft oder mit Silberionen versetzt ausgebildet sein.

Statt oder in Ergänzung zu einer haftenden Beschichtung kann auf der Oberseite, also auf der Außenseite des orthopädischen Interfaces, eine Struktur vorhanden sein, die in Einführrichtung des Interfaces einen geringeren Widerstand als entgegen der Einführrichtung aufweist. Die Struktur kann als ein Strichvelours ausgebildet oder aufgebracht sein, über das eine Kopplung zu einer orthopädischen Baueinheit, beispielsweise einem Außenschaft oder einer Orthesenhalterung, hergestellt werden kann. Dazu ist das Textil oder die Struktur bzw. Beschichtung dergestalt in einer Richtung orientiert geneigt, dass ein Hineingleiten in die Aufnahmeeinrichtung für die Extremität erleichtert wird, ein Herausrutschen jedoch wirksam verhindert werden kann. Zum Lösen der Kopplung zwischen dem Interface und der orthopädischen Baueinheit wird dann die Halterung gelöst, beispielsweise aufgebogen oder aufgeklappt.

Das orthopädische Interface kann als Liner zur Anlage an einen Amputationsstumpf ausgebildet sein, ebenfalls ist es möglich, dass es als flexibler Schaft oder als ein flexibles Schaftteil ausgebildet ist und Aufnahmeeinrichtungen für orthetische oder prothetische Komponenten aufweist.

Zur Erleichterung des Anlegens des orthopädischen Interfaces ist dieses bevorzugt anatomisch vorgeformt, ebenfalls kann es einen offenen Querschnitt aufweisen und über Verschlusseinrichtungen, wie Klettverschlüsse oder dergleichen, angelegt werden.

Das 3D-Textil ist bevorzugt als ein Abstandsgewirk oder als ein Abstandsgestrick ausgebildet. Das Abstandsgewirk weist auf der Oberseite bevorzugt ein Obertextil und auf der Unterseite, also derjenigen Seite, die der Haut des Interfacenutzers zugeordnet ist, ein sogenanntes Untertextil auf. Die Ober- und Untertextilien bilden den Abschluss des Abstandsgewirkes und die Kontaktflächen zu der Orthese oder Prothese einerseits und der Haut andererseits.

Das erfindungsgemäße System sieht ein Interface, wie es voranstehend beschrieben wurde, sowie eine damit gekoppelte Orthesen- oder Protheseneinrichtung vor, die über Kopplungsmittel an dem Obertextil bzw. der Außenseite des Interfaces festgelegt werden kann. Die Orthesen- oder Protheseneinrichtung kann eine im Wesentlichen formstabile Aufnahme mit einer innenseitigen Beschichtung aufweisen, wobei die Beschichtung aus einem haftenden Material, beispielsweise Silikon, Polyurethan oder Copolymer oder einer anderen Beschichtung, beispielsweise Klettverschlusselementen oder einem Strichvelours, ausgebildet sein kann, um eine Koppelung der formstabilen Aufnahme mit dem Interface zu realisieren. Alternativ oder ergänzend kann eine Struktur der Aufnahme auf der dem Interface zugeordneten Seite vorgesehen sein, die einen geringen Einführwiderstand und einen hohen Widerstand entgegen der Einführrichtung aufweist. Die Struktur kann aus entsprechend orientierten Formschlusselemente oder dergleichen aufgebaut sein.

Nachfolgend werden Ausführungsbeispiele der Erfindung anhand der beigefügten Figuren näher erläutert. Es zeigen:
- Figur 1 -: eine perspektivische Darstellung eines Interfaces in Linergestalt;
- Figur 2 -: eine perspektivische Teilansicht einer Protheseneinrichtung;
- Figur 3 -: eine Protheseneinrichtung gemäß Figur 2 mit Interface;
- Figur 4 -: eine vergrößerte Schnittdarstellung durch ein Interface und eine Aufnahme; sowie
- Figur 5 -: eine teilgeschnittene Variante mit Elektroden.

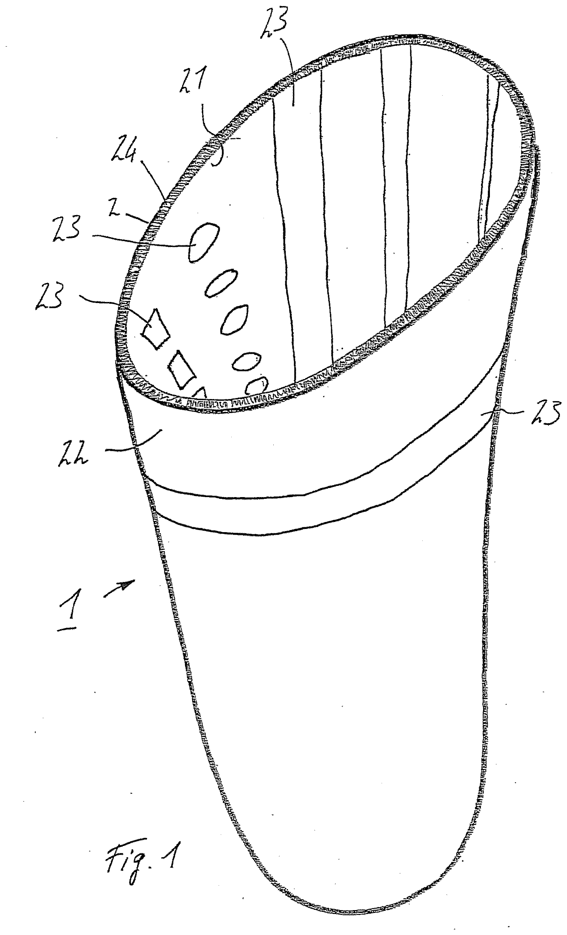

In der Figur 1 ist in einer perspektivischen schematischen Darstellung ein orthopädisches Interface 1 in Gestalt eines sogenannten Liners dargestellt, der anatomisch vorgeformt ist, um an einem Amputationsstumpf angelegt zu werden. Das orthopädische Interface 1 besteht aus einem 3D-Textil, vorliegend in Gestalt eines Abstandsgewirkes 2, das eine der Haut des Interfacenutzers zugewandte Unterseite 21 aus einem Untertextil und einem der Haut abgewandten Obertextil auf der Oberseite 22 besteht, zwischen denen Stützfäden 24 angeordnet sind, die die Ober- und Untertextilien 22, 21 beabstandet zueinander halten und miteinander verbinden. Sofern keine Obertextilien in dem 3D-Textil vorhanden sind, werden die jeweiligen Ober- und Unterseiten mit den Bezugszeichen 22, 21 versehen, die Ober- und Untertextilien 22, 21 entsprechen somit den Ober- und Unterseiten. Zwischen den Ober- und Untertextilien 22, 21 bildet sich durch die Stützfäden 24 ein gut durchlüfteter Zwischenraum aus. Andere 3D-Textilen, beispielsweise Abstandsgestricke, auch ohne Unter- und Obertextilien oder nur mit einem Unter- oder Obertextil, können ebenfalls eingesetzt werden. Das Interface 1 kann aus einem Zuschnitt durch Vernähen hergestellt werden. Ebenfalls ist es möglich, den Zuschnitt auf andere Art und Weise zu verbinden, beispielsweise durch Verkleben oder Verschweißen.

Auf der Innenseite des Interfaces 1 sind Bereiche mit einer haftenden Beschichtung 23 aufgebracht, die auf der Haut des Interfacenutzers, zum Beispiels auf einem Amputationsstumpf, anhaften. Diese Beschichtungen 23 können linienförmig, punktförmig, kreisförmig oder eckig ausgebildet sein. Grundsätzlich ist es ebenfalls möglich, dass die gesamte Innenseite des Liners, also die Oberfläche des Untertextils 21 mit einer Beschichtung 23 versehen ist. Bei einer nur bereichsweisen Beschichtung ist ein verbesserter Feuchtigkeitstransport von der Haut des Nutzers in die Umgebung gegeben, so dass ein erhöhter Tragekomfort realisiert wird, eine vollflächige Beschichtung 23 hat dagegen eine verbesserte Haftung aufgrund der vergrößerten Haftfläche zur Folge.

Als Beschichtung 23 kommen insbesondere Silikonbeschichtungen, Polyurethanbeschichtungen oder Beschichtungen aus einem Copolymer in Frage, grundsätzlich sind jedoch auch andere Beschichtungen vorgesehen und möglich, die ein verbessertes Anhaften des Untertextils 21 und damit der 3D- Textils 1 an der Hautoberfläche bewirken.

An dem Obertextil 22, also an der Außenseite des Liners bzw. des orthopädischen Interfaces, kann ebenfalls eine Beschichtung 23 angeordnet sein. Diese Beschichtung kann ebenfalls aus einem haftenden Material bestehen, wobei die Außenseite des Liners oder des Interfaces 1 mit einer Beschichtung versehen ist, die zu einem darum angeordneten orthopädischen Bauteil, beispielsweise einem Prothesenschaft oder einer Orthesenhalterung ausgewählt ist. Auch hier ist es vorgesehen, dass eine verbesserte Anhaftung des Abstandsgewirkes 2 an dem außen anliegenden Bauteil gewährleistet ist. Auch können formschlüssig wirkende Elemente wie Strichvelours oder Klettverschlussbereiche an dem Obertextil 22 angeordnet oder ausgebildet sein.

Statt eines geschlossenen Querschnittes, wie er in der Figur 1 dargestellt ist, kann das Interface 1 auch einen offenen Querschnitt aufweisen, ebenfalls kann ein teilweise geschlossener und teilweise offener Querschnitt realisiert sein. Ebenfalls ist es möglich, dass Verschlusseinrichtungen an dem Interface 1 angeordnet sind, mit denen ein Öffnen und Schließen zur Erleichterung des An- und Ablegens möglich ist.

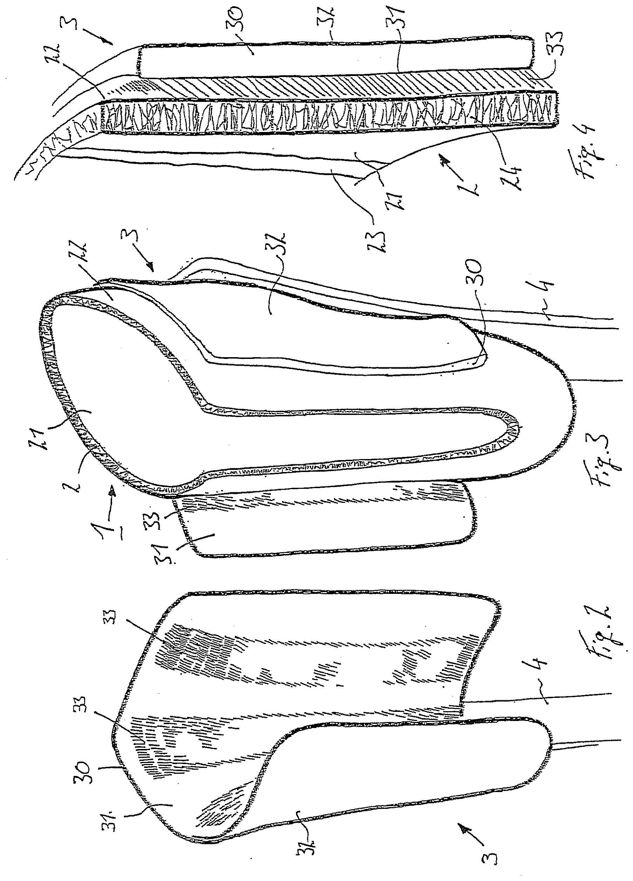

In der Figur 2 ist eine Halterung 3 einer Prothese mit einem Verbindungsschenkel 4 gezeigt, die die Halterung 3 mit einer Gelenkeinrichtung verbindet. Die Halterung 3 weist ein rohrartiges, gekrümmtes Aufnahmeteil 30 mit einem offenen Querschnitt aus einem elastischen, stabilen Kunststoff auf, mit einer Außenseite 32 und einer Innenseite 31. Auf der Innenseite 31 der Halterung 3 sind Beschichtungsbereiche 33 ausgebildet, die entweder aus einem haftenden Material oder aus einem Strichvelours bestehen. Ebenfalls ist es möglich, mehrere Beschichtungen 33 unterschiedlicher Art vorzusehen. Grundsätzlich kann auch die komplette Innenseite 31 der Halterung 3 mit einer Beschichtung versehen sein. Auch können alternative Strukturen auf der Innenseite der Halterung 3 vorhanden sein, die in Einführrichtung des Interfaces einen geringeren Widerstand als entgegen der Einführrichtung bereitstellen, beispielsweise entsprechend geformte Formschlusselemente, die in korrespondierend geformte Strukturen in dem Interface eingreifen.

In der Figur 3 ist die Halterung 3 mit dem Verbindungsschenkel 4 in Zusammenwirkung mit dem orthopädischen Interface 1 dargestellt. In der dargestellten Ausführungsform ist die Beschichtung 33 der Halterung 3 als Strichvelours ausgeführt und in einer Orientierung ausgebildet, dass das orthopädische Interface 1, das als Liner für einen Amputationsstumpf ausgebildet ist, von oben eingeführt werden kann. Der Strich des Strichvelours 33 ist dabei nach unten geneigt orientiert, so dass das orthopädische Interface 1 leicht in die Halterung 3 hineingleiten kann, ein Herausrutschen jedoch nicht möglich ist bzw. erschwert wird. Zum Lösen des orthopädischen Interfaces 1 von der Halterung 3 wird diese aufgebogen. Der offene Querschnitt der Halterung 3 kann durch Riemen oder Spannmittel überbrückt werden, um eine radiale Kompression und Stabilität bereitzustellen. Der teiloffene Aufbau des orthopädischen Interfaces 1 kann ebenfalls durch Verschlusseinrichtungen überbrückt werden.

In der Figur 4 ist in einer vergrößerten Detaildarstellung das Zusammenwirken der Halterung 3 mit dem Abstandsgewirk 2 des orthopädischen Interfaces 1 dargestellt. Auf der Innenseite des orthopädischen Interfaces 1, also auf dem Untertextil 21, ist eine haftende Beschichtung 23, beispielsweise aus Copolymer oder Silikon aufgebracht. Die Beschichtung 23 ist in Längserstreckung orientiert aufgebracht und ermöglicht eine stabile Zuordnung der Hautoberfläche zu dem Interface 1. Über die Stützfäden 24 werden ein Luftpolster und eine elastische Abpolsterung bereitgestellt. Auf der Außenseite des Interfaces 1, also an dem Obertextil 22, ist keine Beschichtung vorgesehen, vielmehr wird eine Kopplung mit der Halterung 3 über den Strichvelours 33 realisiert, der so orientiert ist, dass ein Einführen des orthopädischen Interfaces 1 von oben nach unten ermöglicht ist, ein Herausziehen in Richtung nach oben jedoch erschwert oder unmöglich gemacht wird.

Das orthopädische Interface 1 kann auch als flexibler Schaft oder flexibles Schaftteil eingesetzt werden, ebenfalls kann eine entsprechende Innenbeschichtung für Strümpfe oder andere abstützende Funktionen zwischen einem Körperteil und einer externen Einrichtung oder einem Bekleidungsstück oder einer Funktionseinheit vorgesehen sein, um eine gleichmäßige und großflächige Druckverteilung auf das Körperteil bei präzisier Zuordnung und einem hohen Tragekomfort bereitstellen zu können.

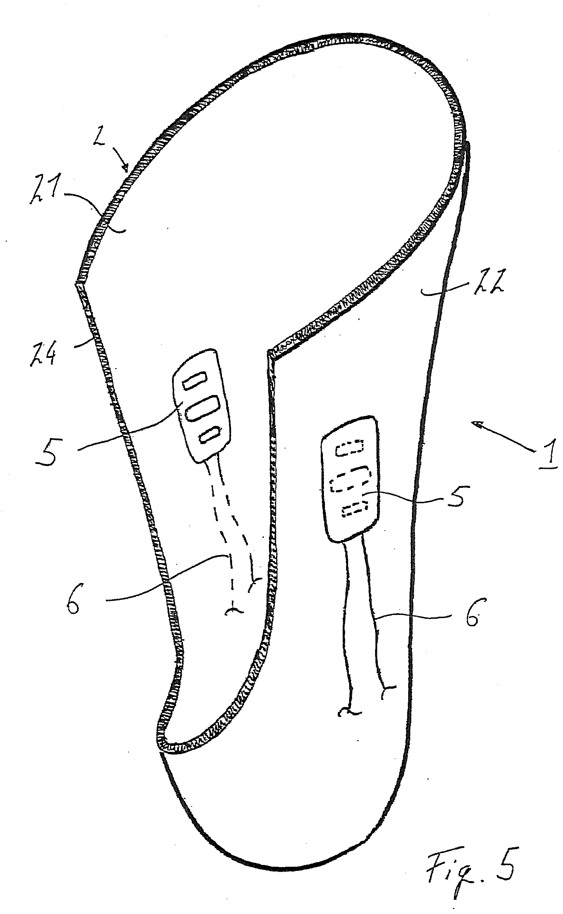

Es hat sich überraschend gezeigt, dass trotz der Luftschicht zwischen der Oberseite und der Unterseite eine gute Koppelung der orthopädischen Einrichtung wie Orthese oder Prothese und dem Interfacenutzer erreicht werden kann, was vorher nicht für möglich gehalten wurde. Auch ein 3D-Textil ohne haftende Beschichtung auf der der Haut zugewandten Seite kann als orthopädisches Interface eingesetzt werden und weist einen überraschend hohen Tragekomfort bei gleichzeitiger Tragsicherheit auf. In der Figur 5 ist eine Variante der Erfindung gezeigt, bei der das orthopädische Interface 1 in einer Teilschnittdarstellung gezeigt ist. Der Aufbau des 3D-Textils 2 entspricht dem der Figuren 1, 3 und 4, die gegebenenfalls vorhandenen Beschichtungen auf der Innen- oder Außenseite 21, 22 sind nicht dargestellt. Innerhalb des orthopädischen Interfaces sind zwei Elektroden 5 angeordnet, die über elektrische Anschlussmittel 6 Daten an eine nicht dargestellte Auswerteeinheit übermitteln können. Ebenfalls ist es möglich, eine kabellose Datenübertragung von den Elektroden 5 vorzunehmen. Die Elektroden 5 sind auf der Innenseite 21 des Abstandsgewirkes 2 angeordnet und können dort aufgenäht, aufgeklebt, aufgeschweißt oder anderweitig daran befestigt sein. Über diese Elektroden 5 werden Oberflächenpotentiale abgeleitet, so dass beispielsweise über myoelektrische Signale eine Prothesenansteuerung erfolgen kann. Ebenfalls ist es möglich, dass die Elektroden 5 innerhalb des Abstandsgewirkes 2 integriert sind, so dass nur die Kontaktflächen der Elektroden 5 mit der Hautoberfläche über die Innenseite 21 des 3D-Textils 2 hinausstehen. Eine Durchführung der elektrischen Anschlussmittel 6 kann durch Ausnehmungen innerhalb des 3D-Textils 2, das z.B. als Abstandsgewirk oder Abstandsgestrick ausgebildet ist, erfolgen. Ebenfalls kann zur Befestigung der Elektroden ein Fenster aus dem Abstandsgewirk oder Abstandsgestrick 2 herausgeschnitten und die Elektrode in das Fenster eingesetzt werden.

## Patentansprüche

1. Orthopädisches Interface mit einem flächigen 3D-Textil mit einer Oberseite (22) und einer Unterseite (21), die über Stützfäden zueinander beabstandet gehalten und miteinander verbunden sind, wobei die Unterseite (21) des 3D-Textils zur Auflage auf der Haut eines Interfacenutzers ausgebildet ist, wobei die der Haut zugeordnete Oberfläche der Unterseite (21) zumindest teilweise mit einer haftenden Beschichtung (23) versehen ist, **dadurch gekennzeichnet, dass** die Beschichtung (23) elektrisch leitfähig ist und/oder Elektroden (5) auf der Unterseite (21) aufgebracht oder in dem 3D-Textil (2) eingearbeitet sind.

2. Orthopädisches Interface nach Anspruch 1, **dadurch gekennzeichnet, dass** die der Haut abgewandte Oberseite (22) zumindest teilweise mit einer Beschichtung (23) versehen ist.

3. Orthopädisches Interface nach Anspruch 1 oder 2, **dadurch gekennzeichnet, dass** die Beschichtung (23) aus Silikon, Polyurethan und/oder einem Copolymer besteht.

4. Orthopädisches Interface nach einem der voranstehenden Ansprüche, **dadurch gekennzeichnet, dass** die Beschichtung (23) in zueinander beabstandeten Bereichen, insbesondere streifenförmig, punktförmig, ringförmig oder in anderen Formen oder vollflächig aufgebracht ist.

5. Orthopädisches Interface nach einem der voranstehenden Ansprüche, **dadurch gekennzeichnet, dass** die Beschichtung (23) luftdurchlässig und/oder feuchtigkeitsdurchlässig ausgebildet ist.

6. Orthopädisches Interface nach einem der voranstehenden Ansprüche, **dadurch gekennzeichnet, dass** die verarbeiteten Fäden des 3D-Textils (2) zumindest teilweise beschichtet sind.

7. Orthopädisches Interface nach einem der voranstehenden Ansprüche, **dadurch gekennzeichnet, dass** in dem 3D-Textil (2), insbesondere in der Unterseite (21), Silberfäden eingewebt oder eingesponnen sind.

8. Orthopädisches Interface nach einem der voranstehenden Ansprüche, **dadurch gekennzeichnet, dass** die Oberseite (22) eine Struktur aufweist, die in Einführrichtung des Interfaces (1) einen geringeren Widerstand als entgegen der Einführrichtung aufweist.

9. Orthopädisches Interface nach einem der voranstehenden Ansprüche, **dadurch gekennzeichnet, dass** es als Liner zur Anlage an einen Amputationsstumpf ausgebildet ist.

10. Orthopädisches Interface nach einem der Ansprüche 1 bis 7, **dadurch gekennzeichnet, dass** es als flexibler Schaft oder als flexibles Schaftteil mit Aufnahmeeinrichtungen für orthetische oder prothetische Komponenten ausgebildet ist.

11. Orthopädisches Interface nach einem der voranstehenden Ansprüche, **dadurch gekennzeichnet, dass** das 3D-Textil als Abstandsgestrick oder Abstandsgewirk ausgebildet ist.

12. Orthopädisches Interface nach einem der voranstehenden Ansprüche, **dadurch gekennzeichnet, dass** die Unterseite (21) als ein Untertextil und die Oberseite (22) als ein Obertextil ausgebildet ist.

13. System aus einem orthopädischen Interface (1) nach einem der voranstehenden Ansprüche und einer Orthesen- oder Protheseneinrichtung (3, 4).

14. System nach Anspruch 13, **dadurch gekennzeichnet, dass** die Prothesen- oder Ortheseneinrichtung (3, 4) eine im Wesentlichen formstabile Aufnahme (13) mit einer Struktur aufweist, die in Einführrichtung des Interfaces (1) einen geringeren Widerstand als entgegen der Einführrichtung aufweist.

## Claims

1. Orthopedic interface comprising a planar 3D textile with a top (22) and a bottom (21) that are held at a distance from each other by supporting threads and connected to each other, the bottom (21) of the 3D textile being designed to bear on the skin of an interface user, wherein the surface of the bottom (21) being assigned to the skin is provided at least partially with an adhesive coating (23), **characterized in that** the coating (23) is electrically conductive and/or electrodes (5) are applied to the bottom (21) or worked into the 3D textile (2).

2. Orthopedic interface as claimed in claim 1, **characterized in that** the top (22) facing away from the skin is provided at least partially with a coating (23).

3. Orthopedic interface as claimed in claim 1 or 2, **characterized in that** the coating (23) is composed of silicone, polyurethane and/or a copolymer.

4. Orthopedic interface as claimed in one of the preceding claims, **characterized in that** the coating (23) is applied in areas set apart from one another, in particular in the shape of strips, dots, rings, or in other shapes or over the whole surface.

5. Orthopedic interface as claimed in one of the preceding claims, **characterized in that** the coating (23) is air-permeable and/or moisture-permeable.

6. Orthopedic interface as claimed in one of the preceding claims, **characterized in that** the threads worked into the 3D textile (2) are at least partially coated.

7. Orthopedic interface as claimed in one of the preceding claims, **characterized in that** silver threads are woven or spun into the 3D textile (2), particularly at the bottom (21).

8. Orthopedic interface as claimed in one of the preceding claims, **characterized in that** the top (22) has a structure that has less resistance in the direction of insertion of the interface (1) than counter to the direction of insertion.

9. Orthopedic interface as claimed in one of the preceding claims, **characterized in that** it is designed as a liner for bearing on an amputation stump.

10. Orthopedic interface as claimed in one of the claims 1 to 7, **characterized in that** it is designed as a flexible socket or as a flexible socket part with receiving means for orthotic or prosthetic components.

11. Orthopedic interface as claimed in one of the preceding claims, **characterized in that** the 3D textile is designed as a spaced knitted fabric or as a spacer fabric.

12. Orthopedic interface as claimed in one of the preceding claims, **characterized in that** the bottom (21) is designed as a lower textile and the top (22) is designed as an upper textile.

13. System composed of an orthopedic interface (1) as claimed in one of the preceding claims and of an orthotic or prosthetic device (3, 4).

14. System as claimed in claim 13, **characterized in that** the prosthetic or orthotic device (3, 4) has a substantially dimensionally stable receiver (13) with a structure that has less resistance in the direction of insertion of the interface (1) than counter to the direction of insertion.

## Revendications

1. Interface orthopédique comprenant un textile surfacique 3D présentant une face supérieure (22) et une face inférieure (21) qui sont maintenues écartées l'une de l'autre et reliées l'une à l'autre via des filaments de soutien, dans laquelle la face inférieure (21) du textile 3D est réalisée pour venir en contact avec la peau d'un utilisateur de l'interface, dans laquelle la surface, tournée vers la peau, de la face inférieure (21) est dotée au moins partiellement d'un revêtement adhésif (23),
**caractérisée en ce que** le revêtement (23) est conducteur de l'électricité et/ou **en ce que** des électrodes (5) sont appliquées sur la face inférieure (21) ou sont intégrées dans le textile 3D (2).

2. Interface orthopédique selon la revendication 1, **caractérisée en ce que** la face supérieure (22), détournée de la peau, est au moins partiellement dotée d'un revêtement (23).

3. Interface orthopédique selon la revendication 1 ou 2, **caractérisée en ce que** le revêtement (23) est en silicone, en polyuréthane et/ou en un copolymère.

4. Interface orthopédique selon l'une des revendications précédentes, **caractérisée en ce que**, dans des zones écartées les unes des autres, le revêtement (23) est appliqué en particulier sous forme de rubans, sous forme de points, sous forme d'anneaux ou d'autres conformations, ou encore appliqué sur toute la surface.

5. Interface orthopédique selon l'une des revendications précédentes, **caractérisée en ce que** le revêtement (23) est réalisé de manière perméable à l'air et/ou perméable à l'humidité.

6. Interface orthopédique selon l'une des revendications précédentes, **caractérisée en ce que** les filaments traités du textile 3D (2) sont au moins partiellement revêtus.

7. Interface orthopédique selon l'une des revendications précédentes, **caractérisée en ce que** des fils d'argent sont tissés ou enfilés dans le textile 3D (2), en particulier dans la face inférieure (21).

8. Interface orthopédique selon l'une des revendications précédentes, **caractérisée en ce que** la face supérieure (22) présente une structure qui présente dans la direction d'introduction de l'interface (1) une résistance plus faible qu'en sens contraire à la direction d'introduction.

9. Interface orthopédique selon l'une des revendications précédentes, **caractérisée en ce qu'**elle est réalisée à titre de doublage pour l'application sur un moignon d'amputation.

10. Interface orthopédique selon l'une des revendications 1 à 7, **caractérisée en ce qu'**elle est réalisée sous forme d'emboîture flexible ou de partie d'emboîture flexible avec des moyens de réception pour des composants orthétiques ou prothétiques.

11. Interface orthopédique selon l'une des revendications précédentes, **caractérisée en ce que** le textile 3D est réalisé sous forme de tricot ou de non-tissé d'écartement.

12. Interface orthopédique selon l'une des revendications précédentes, **caractérisée en ce que** la face inférieure (21) est réalisée sous forme de textile inférieur et la face supérieure (22) est réalisée sous forme de textile supérieur.

13. Système constitué d'une interface orthopédique (1) selon l'une des revendications précédentes, et d'un dispositif formant orthèse ou d'un dispositif formant prothèse (3, 4).

14. Système selon la revendication 13, **caractérisé en ce que** le dispositif formant prothèse ou le dispositif formant orthèse (3, 4) comporte un logement (13) de forme essentiellement stable avec une structure qui présente dans la direction d'introduction de l'interface (1) une résistance plus faible qu'en sens contraire à la direction d'introduction.
